# EUROPEAN PATENT APPLICATION

(11) **EP 4 226 865 A1**
(43) Date of publication of application: **16.08.2023**
(21) Application number: 21877794.4
(22) Date of filing: 19.03.2021
(51) Int. Cl.: A61B 10/00, A61B 5/08, A61B 5/145, A61B 5/157, G01N 33/569, B01L 3/00

(54) **DIAGNOSTIC KIT IN WHICH DETECTION DEVICE AND SAMPLING TOOL ARE INTEGRATED**

(30) Priority: 08.10.2020 KR 20200130477; 17.02.2021 KR 20210021139
(71) Applicant: Bionote, Inc., Hwaseong-si, Gyeonggi-do 18449 (KR); SD Biosensor, Inc., Gyeonggi-do 16690 (KR)
(72) Inventor: CHO, Young Shik, Yongin-si, Gyeonggi-do 16951 (KR); KIM, Jung Ho, Hwaseong-si, Gyeonggi-do 18477 (KR); PARK, Hyo Lim, Suwon-si, Gyeonggi-do 16685 (KR); KIM, Dong Hyuk, Ansan-si, Gyeonggi-do 15594 (KR)
(74) Representative: Studio Torta S.p.A.
(86) International application number: PCT/KR2021/003416
(87) International publication number: WO 2022/075539

(57) **Abstract**

Provided is a diagnostic kit, in which a detection device and a specimen collection tool are integrated. The diagnostic kit includes a specimen collector capable of collecting a specimen, a device including a housing and a detection strip, and a connector connecting the specimen collector with the device and moving a solution, the connector having a bar shape.

## Description

### Technical Field

The present disclosure relates to a diagnostic kit, in which a detection device and a specimen collection tool are integrated, and more particularly, to a diagnostic kit, in which a detection device and a specimen collection tool are integrated, for consecutively performing a specimen collection process and a testing process without separation therebetween by integrating the detection device with the specimen collection tool that may be used for respiratory infection tests or coronavirus tests.

### Background Art

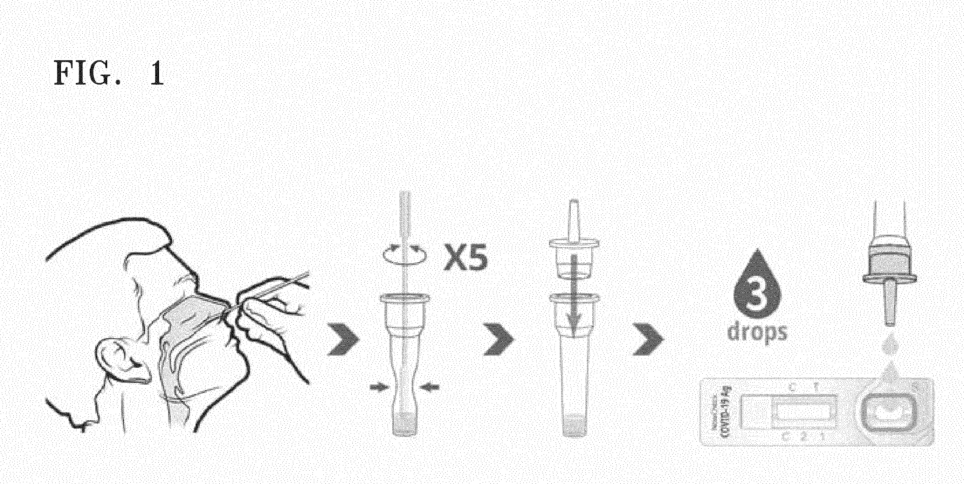

In general, respiratory infection tests or coronavirus tests are performed by collecting specimens such as saliva or nasal mucus and testing the specimens. FIG. 1 is a diagram illustrating a diagnostic kit according to the related art and a testing method using the diagnostic kit. Referring to FIG. 1, in the diagnostic kit according to the related art, a specimen collection tool for collecting a specimen is separated from a detection device for performing a test.

In a testing process using the diagnostic kit according to the related art, a specimen is collected by the specimen collection tool, and then, the specimen collection tool with the collected specimen is inserted into a tube containing a developing solution. A target material of the specimen is extracted in the tube containing the developing solution and then dripped onto the detection device that is separated, thereby performing a test.

However, a method of performing a test by using the diagnostic kit according to the related art has issues as follows. In the method of performing a test by using the diagnostic kit according to the related art, because the specimen collection tool is separated from the detection device, a specimen collection process and a testing process are not consecutive and are separated from each other.

When the specimen collection process and the testing process are separated from each other as such, because there is an increasing possibility that a specimen could be exposed to other contaminant sources during the process of testing, there is an issue in that it is difficult to accurately perform a test. In particular, during the process in which the specimen collected by the specimen collection tool is extracted by a diluent and then moved to the detection device, there is an increasing possibility that the specimen could be exposed to contaminant sources.

In addition, the specimen collection tool having collected the specimen is contained in the tube containing the developing solution and then discarded separately from the detection device, and during such a process, there is an issue in that an infection source remaining on the specimen collection tool may risk being leaked to the outside thereof.

### Disclosure

### Technical Problem

To solve the issues set forth above, the present disclosure provides a diagnostic kit, in which a detection device and a specimen collection tool are integrated, for consecutively performing a specimen collection process and a testing process without separation therebetween by integrating the detection device with the specimen collection tool that may be used for respiratory infection tests or coronavirus tests.

### Technical Solution

According to an aspect of the present disclosure, a diagnostic kit, in which a detection device and a specimen collection tool are integrated, for diagnosing a target material includes a specimen collector capable of collecting a specimen, a device including a housing and a detection strip, and a connector connecting the specimen collector with the device and moving a solution, the connector having a bar shape.

In the diagnostic kit, the specimen may include a discharge inside a nasal cavity, and the diagnostic kit may be used for a respiratory infection test or a coronavirus test.

In the diagnostic kit, a head portion of the specimen collector may include a sponge material.

In the diagnostic kit, an opposite portion of the specimen collector may be open to fit into the connector, and the width of the opposite portion of the specimen collector may be less than the width of the connector.

In the diagnostic kit, an elastic body may be arranged on the opposite portion of the specimen collector.

In the diagnostic kit, the connector may include a porous material, through which a solution is allowed to move.

In the diagnostic kit, the connector may include a catching protrusion, which protrudes outward from the connector.

In the diagnostic kit, a catching groove, into which the catching protrusion is allowed to fit, is arranged inside the housing at one side of the housing.

In the diagnostic kit, the housing may include a support having a bar shape at one end thereof, the support extending from the one end of the housing to the outside of the housing and being capable of supporting the connector while contacting the outside of the connector.

In the diagnostic kit, when the specimen collector is inserted into a tube storing a developing solution, a test may be performed by the device connected with the specimen collector via the connector.

In the diagnostic kit, the housing may include an insertion portion at one side thereof, the insertion portion having a cross-section of a polygonal shape.

In the diagnostic kit, the detection strip of the device may include a sample pad absorbing a sample, a conjugation pad located under the sample pad and including a source material that reacts with a target material, and a membrane including a test line coated with the source material reacting with the target material and a control line coated with a source material used for a control group.

In the diagnostic kit, the source material may be coupled with nano-sized nanoparticles and impregnated into the conjugation pad, and the nanoparticles may include one or more of gold particles, silver particles, latex, cellulose, and fluorescent particles.

In the diagnostic kit, the conjugation pad may include polyester or glass fiber.

In the diagnostic kit, the diagnostic kit may further include an absorption pad capable of absorbing the sample that does not react in the conjugation pad and the membrane.

In the diagnostic kit, the target material may include a material extracted from a coronavirus or a material extracted from a respiratory infection source.

### Advantageous Effects

The present disclosure relates to a diagnostic kit, in which a detection device and a specimen collection tool are integrated, and the diagnostic kit has a merit in that a specimen collection process and a testing process may be consecutively performed without separation therebetween by integrating a detection device with a specimen collection tool that may be used for respiratory tests or coronavirus tests.

In this way, the present disclosure has a merit of providing a diagnostic kit that may increase consumer convenience and allow a quick test by reducing a testing operation. In addition, the present disclosure has a merit of cutting off the possibility that an infection source could be leaked from the specimen collection tool because the detection device and the specimen collection tool are discarded together, and also has a merit of reducing the possibility that the specimen could be exposed to contaminant sources during the process of testing.

Furthermore, the present disclosure has a merit in that, because a connector includes a catching protrusion and a housing includes a catching groove to allow the catching protrusion to fit into the catching groove, the flow of a solution moving along the connector may be facilitated.

### Description of Drawings

FIG. 1 is a diagram illustrating that a test is performed by using a diagnostic kit according to the related art.
FIG. 2 is a diagram illustrating that a test is performed by using a diagnostic kit, in which a detection device and a specimen collection tool are integrated, according to an embodiment of the present disclosure.
FIG. 3 is a perspective view of a diagnostic kit, in which a detection device and a specimen collection tool are integrated, according to an embodiment of the present disclosure.
FIG. 4 is an exploded perspective view of a diagnostic kit, in which a detection device and a specimen collection tool are integrated, according to an embodiment of the present disclosure.
FIG. 5 is a diagram illustrating a detection strip according to an embodiment of the present disclosure.
FIG. 6 is a diagram illustrating that a plurality of test lines and control lines are arranged in a detection strip according to an embodiment of the present disclosure.
FIG. 7 is a diagram illustrating that a source material is coupled with nanoparticles and then reacts with a target material, according to an embodiment of the present disclosure.
FIG. 8 is a diagram illustrating that a connector includes a catching protrusion and a housing includes a catching groove into which the catching protrusion may fit, according to an embodiment of the present disclosure.

### Mode for Invention

The specification describes the principle of the present disclosure and embodiments to clarify the scope of the present disclosure and allow those of ordinary skill in the art to implement the present disclosure. Disclosed embodiments may be implemented in various ways.

As used herein, the terms such as "includes (or comprises)" or "may include (or comprise)" indicate the presence of a function, an operation, a component, or the like, which is disclosed, and do not limit additional one or more functions, operations, components, or the like. In addition, it should be understood that the terms such as "includes (or comprises)" or "has" used herein specify the presence of stated features, numbers, steps, operations, components, parts, or combinations thereof, but do not preclude the presence or addition of one or more other features, numbers, steps, operations, components, parts, or combinations thereof.

Herein, it should be understood that, when one component is referred to as being "coupled to" or "connected to" another component, the one component may be directly coupled to or directly connected to the other component or may be coupled to or connected to the other component with an intervening component therebetween, unless otherwise stated. On the other hand, it should be understood that, when one component is referred to as being "directly coupled to" or "directly connected to" another component, there are no intervening components between the one component and the other component.

It will also be understood that, although the terms such as "first", "second" and the like may be used herein to describe various components, these components should not be limited by these terms. These terms are used only to distinguish one component from another component.

The present disclosure relates to a diagnostic kit, in which a detection device and a specimen collection tool are integrated, and more particularly, to a diagnostic kit, in which a detection device and a specimen collection tool are integrated, for consecutively performing a specimen collection process and a testing process without separation therebetween by integrating the detection device with the specimen collection tool that may be used for respiratory infection tests or coronavirus tests.

The diagnostic kit, in which the detection device and the specimen collection tool are integrated, according to an embodiment of the present disclosure may be used for respiratory infection tests or coronavirus tests. However, the diagnostic kit, in which the detection device and the specimen collection tool are integrated, according to an embodiment of the present disclosure may also be expansively used for infectious disease tests, antibody tests, hormone tests, drug tests, and the like.

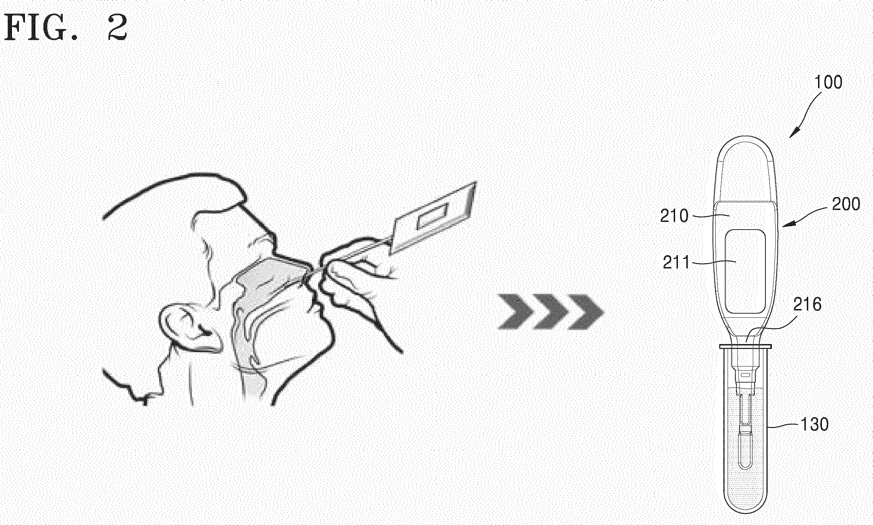

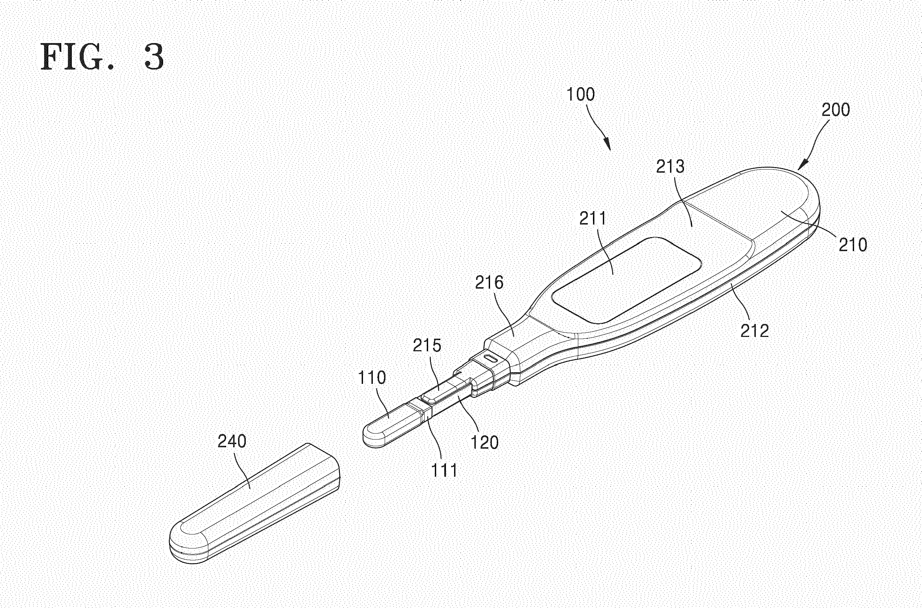

Referring to FIGS. 2 and 3, a diagnostic kit 100, in which a detection device and a specimen collection tool are integrated, according to an embodiment of the present disclosure includes a specimen collector 110, a connector 120, and a device 200.

The specimen collector 110 may collect a specimen, and the specimen collected by the specimen collector 110 may include a discharge inside a nasal cavity. The diagnostic kit 100, in which a detection device and a specimen collection tool are integrated, according to an embodiment of the present disclosure may be used for respiratory infection tests or coronavirus tests, and for this purpose, the specimen collector 110 may collect, as a specimen, a discharge inside a nasal cavity.

However, the diagnostic kit 100, in which a detection device and a specimen collection tool are integrated, according to an embodiment of the present disclosure may also be expansively used for infectious disease tests, antibody tests, hormone tests, drug tests, and the like, and in this case, the specimen may include one of urine, feces, tears, blood, saliva, and nasal mucus.

The specimen collector 110 may have a rod shape extending in a length direction thereof, and a head portion of the specimen collector 110 may include a sponge material. The head portion of the specimen collector 110 is a portion that is in direct contact with the specimen, and thus, a portion that is in direct contact with the nasal cavity.

The head portion of the specimen collector 110 may include a sponge material to collect the specimen without irritating a nasal mucous membrane. However, the head portion of the specimen collector 110 is not limited to a sponge material and may include one or more of a porous polymer, sponge, paper, cotton, cotton wool, and wool, as needed.

The device 200 includes a housing 210 and a detection strip 220, and the device 200 is a place in which a test is performed on a target material.

The connector 120 has a bar shape and connects the device 200 with the specimen collector 110. Because the device 200 is connected with the specimen collector 110 via the connector 120, the specimen collector 110, the connector 120, and the device 200 may be integrated into one body, and thus, a specimen collection process and a testing process may be consecutively performed without separation therebetween, as shown in FIG. 2.

More specifically, the specimen collector 110, the connector 120, and the detection strip 220 of the device 200 may be continuously connected to each other in the stated order, and the connector 120 may include a porous material through which a solution may move, instead of including a solid material.

The connector 120 may include a hydrophilic porous material allowing a specimen solution to be transferred well to a detection device according to capillarity, and the specimen collector 110 including a sponge material may be formed in an outer protrusion of the connector 120. The connector 120 may have various specifications depending on sizes of diagnostic kits.

The target material intended to be identified may move to the detection strip 220 through the connector 120 including a porous material, and thus, a specimen collection process and a testing process may be consecutively performed without separation therebetween.

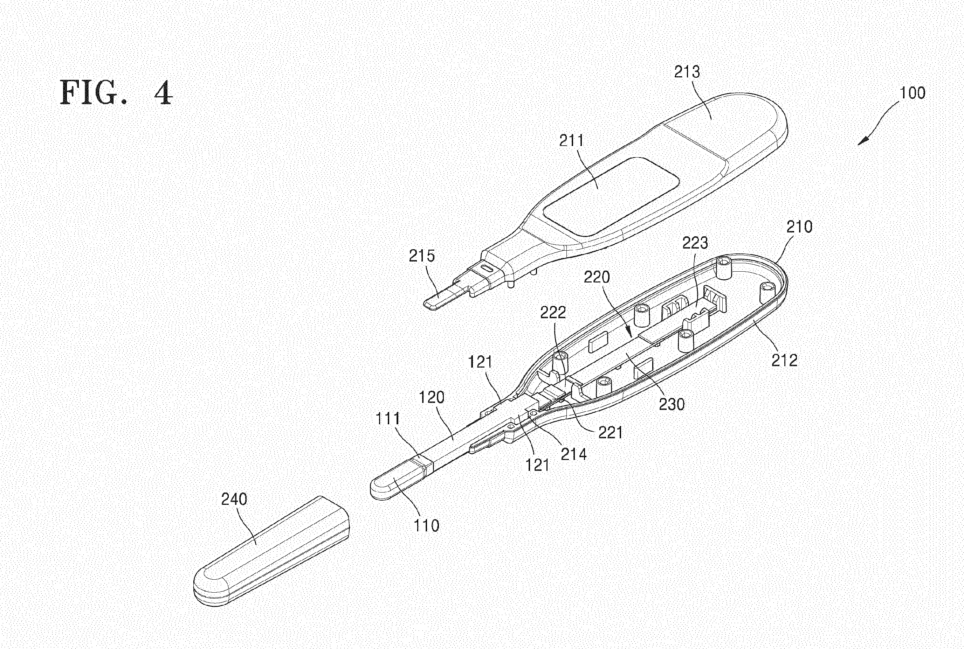

Referring to FIGS. 3 and 4, an opposite portion of the specimen collector 110 with respect to the head portion thereof may be open to fit into the connector 120. The opposite portion of the specimen collector 110 is open to communicate with the outside of the specimen collector 110, and an empty space may be formed in the specimen collector 110.

The connector 120 may be inserted into the empty space in the specimen collector 110 through the open opposite portion of the specimen collector 110, whereby the specimen collector 110 may fit into the connector 120.

Here, a one-side portion of the specimen collector 110 may be a left portion thereof based on FIGS. 3 and 4, and an opposite portion of the specimen collector 110 may be a right portion thereof based on FIGS. 3 and 4.

In addition, in the following description, a one-side portion of each of the connector 120 and the housing 210 may refer to a left portion thereof based on FIGS. 3 and 4, and an opposite portion of each of the connector 120 and the housing 210 may refer to a right portion thereof based on FIGS. 3 and 4.

According to an embodiment of the present disclosure, the width of the opposite portion of the specimen collector 110 may be less than the width of the connector 120. After the specimen collector 110 fits into the connector 120, the specimen collector 110 is required not to depart from the connector 120.

For this purpose, by causing the width of the opposite portion of the specimen collector 110 to be less than the width of the connector 120, the specimen collector 110 may tightly fit into the connector 120.

To tightly fit the specimen collector 110 into the connector 120, the opposite portion of the specimen collector 110 may include an elastic material, or an elastic body 111 such as a rubber band may be arranged on the opposite portion of the specimen collector 110.

As the opposite portion of the specimen collector 110 includes an elastic material or the elastic body 111 is arranged on the opposite portion of the specimen collector 110, the specimen collector 110 may easily fit into the connector 120.

By fitting the specimen collector 110 into the connector 120 in the manner described above, the specimen collector 110 may be prevented from departing from the connector 120.

The device 200 may include various components so long as a test may be performed on the target material, and according to an embodiment of the present disclosure, the device 200 includes the housing 210 and the detection strip 220.

The housing 210 includes a display window 211 that is open to the outside the housing 210. A test line 231 and a control line 232, which are described below, may be located under the display window 211, and a user may check a test result through the display window 211.

Referring to FIGS. 4, 5, and 6, the detection strip 220 may include a sample pad 221, a conjugation pad 222, and a membrane 230.

The sample pad 221 may absorb a sample (specimen solution), and the sample may be a material moving from the connector 120. The sample pad 221 may be made dry to absorb the sample well.

The conjugation pad 222 is located under the sample pad 221 and includes a source material reacting with the target material. The sample absorbed by the sample pad 221 moves to the conjugation pad 222, and when there is the target material in the sample, the sample reacts with the source material of the conjugation pad 222.

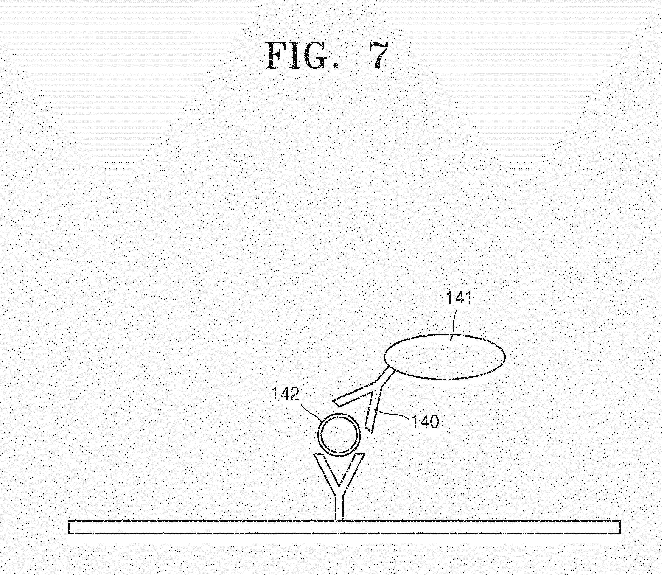

Referring to FIG. 7, a source material 140, which is included in the conjugation pad 222 and reacts with a target material 142, may be coupled with nano-sized nanoparticles 141 and thus impregnated into the conjugation pad 222.

Here, the conjugation pad 222 may include polyester or glass fiber, and the nanoparticles 141 may include one or more of gold particles, silver particles, latex, cellulose, and fluorescent particles. The source material 140 coupled with the nanoparticles 141 is impregnated into polyester or glass fiber and then dried, whereby the conjugation pad 222 including the source material 140 may be fabricated.

Referring to FIG. 7, the target material 142 may be a material extracted from coronavirus or a material extracted from a respiratory infection source. The target material 142 may be a specific antigen of a respiratory pathogen or a specific antigen of coronavirus. In addition, the source material 140 may be a monoclonal anti-coronavirus antibody, which reacts with the target material 142, or an antibody against the respiratory pathogen.

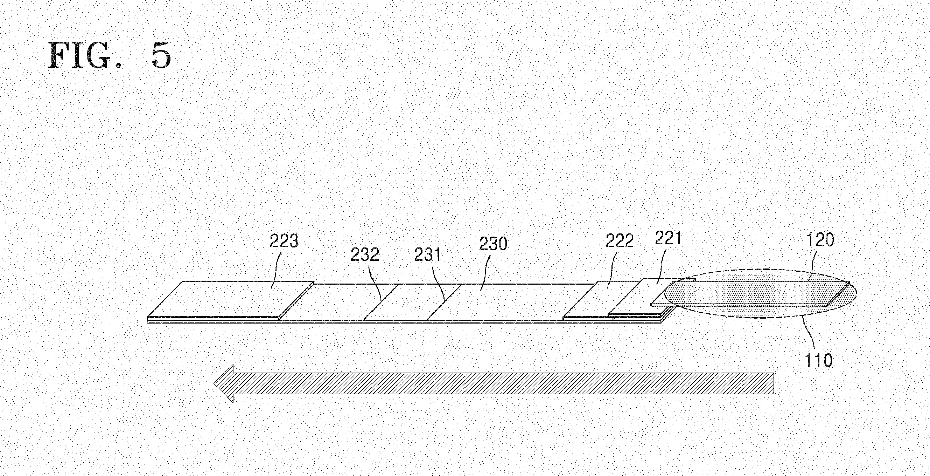

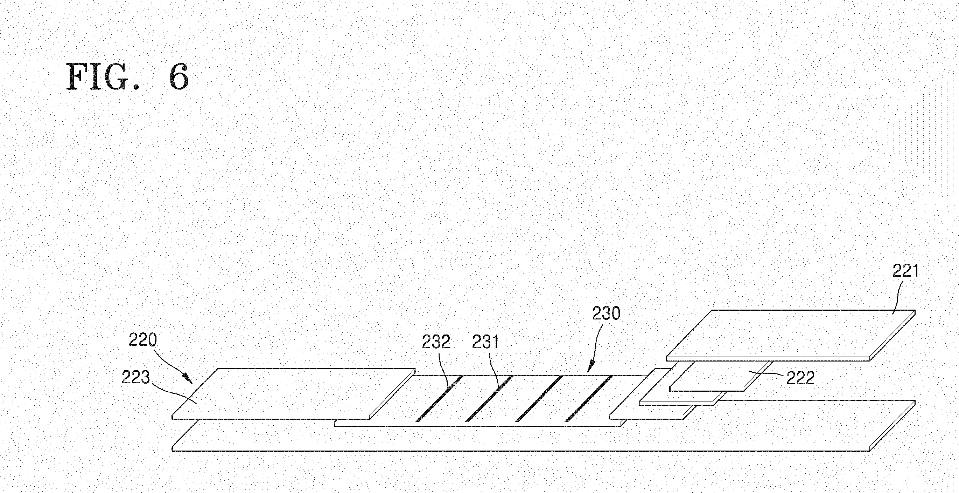

Referring to FIGS. 5 and 6, the membrane 230 may include a test line 231, which is coated with the source material reacting with the target material, and a control line 232, which is coated with a source material used for a control group. The control line 232 may include a visible band, and the test line 231 may be changed into a visible band by a reaction.

The membrane 230 may include nitrocellulose. In addition, the source material reacting with the target material is coated at a position of the test line 231 and then dried, and the source material used for the control group is coated at a position of the control line 232 and then dried, thereby fabricating the membrane 230. The material of the membrane 230 is not limited to nitrocellulose, and other materials may be used for the membrane 230, as needed.

When there is the target material in the sample of the sample pad 221, the source material of the conjugation pad 222 is coupled with the target material first, and then, the coupled product moves along the membrane 230 according to the principle of immunochromatography.

The coupled product having moved to the membrane 230 meets and is coupled, second, with the source material specifically reacting with the target material, at the position of the test line 231. When the second coupling occurs at the position of the test line 231, a visible band at the position of the test line 231 is displayed together with the control line 232, and a user may recognize the visible band.

Referring to FIG. 6, each of the conjugation pad 222 and the test line 232 may be provided in a plural number. A plurality of conjugation pads 222 may be stacked under the sample pad 221 and may respectively include source materials that may be coupled with different target materials from each other.

The membrane 230 may include a plurality of test lines 231 in correspondence with the plurality of conjugation pads 222, and the source materials reacting with the different target materials from each other may be coated at respective test line positions and then dried, thereby forming the plurality of test lines 231.

The detection strip 220 according to an embodiment of the present disclosure may further include an absorption pad 223 capable of absorbing the sample that does not react in the conjugation pad 222 and the membrane 230.

Referring to FIGS. 5 and 6, the absorption pad 223 may absorb the sample that is in contact with the absorption pad 223 in the vicinity of the control line 232 of the membrane 230 and does not react in the conjugation pad 222 and the membrane 230. The absorption pad 223 may include, but is not limited to, a cellulose material, and the absorption pad 223 may be made dry to absorb the sample well.

The sample pad 221, the conjugation pad 222, the membrane 230, and the absorption pad 223, which are fabricated in the methods described above, are coupled with each other to overlap each other while arranged in the stated order from one side to the other. Next, the sample pad 221, the conjugation pad 222, the membrane 230, and the absorption pad 223, which are coupled, are cut into a certain size and arranged in the housing 210.

Specifically, referring to FIG. 4, the housing 210 may include a lower plate 212 and an upper plate 213, and the sample pad 221, the conjugation pad 222, the membrane 230, and the absorption pad 223, which are coupled to overlap each other, are cut and then arranged on the lower plate 212 of the housing 210. Here, the test line 231 and the control line 232 of the membrane 230 need to be located under the display window 211 of the housing 210.

Next, the specimen collector 110 and the connector 120 are aligned and coupled with an insertion hole formed in the lower plate 212 of the housing 210, followed by covering the specimen collector 110 and the connector 120 with the upper plate 213 of the housing 210, thereby fabricating the diagnostic kit 100 according to an embodiment of the present disclosure, as shown in FIG. 3.

Referring to FIG. 4, when the specimen collector 110 and the connector 120 are aligned and coupled with the insertion hole formed in the lower plate 212 of the housing 210, the connector 120 may be stacked on and coupled with the detection strip 220. Specifically, the connector 120 may be stacked on and secured to the sample pad 221 of the detection strip 220.

When the connector 120 is coupled with the detection strip 220, the use of an electrochemical fusion method has issues of difficult fabrication and poor yield. Therefore, the connector 120 may be coupled with the detection strip 220 by a physical method.

When the connector 120 is coupled with the detection strip 220 by a physical method, because the use of a straight gripper pin may hinder a development rate or flow, the connector 120 may be coupled with the detection strip 220 via a pin.

Here, the pin may include a circular pin rather than an angular pin, such as a quadrangular pin. Because an angular pin, such as a quadrangular pin, may generate the bias of flow of the sample, the connector 120 may be secured to the detection strip 220 via a circular pin.

Specifically, a circular pin may be arranged on the housing 210, and a hole may be formed in the connector 120. By fitting the circular pin into the hole of the connector 120, the connector 120 may be secured to the detection strip 220.

Each of the diameter of the circular pin and the diameter of the hole of the connector 120 may be, but is not limited to, 0.5 mm, and may vary depending on specifications of diagnostic kits.

According to another embodiment of the present disclosure, the connector 120 may be coupled with the housing 210 without the use of a pin. Even when the pin includes a circular pin, the flow of the sample moving along the connector 120 may not be facilitated due to the pin. To prevent such an issue, the connector 120 may include a catching protrusion 121 protruding outward from the connector 120.

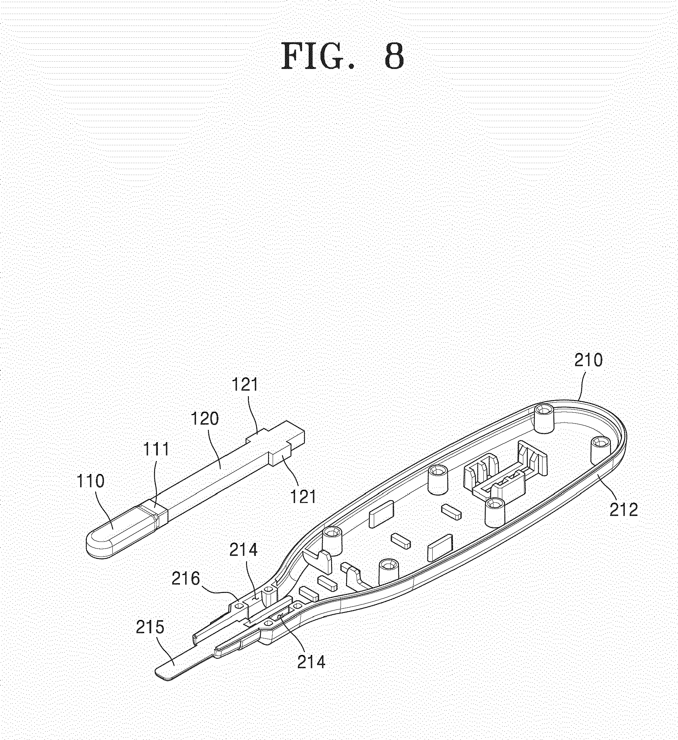

Referring to FIG. 8, the catching protrusion 121 protrudes from both sides of the connector 120 having a bar shape. As the catching protrusion 121 is formed on the connector 120, the connector 120 may have a cross shape.

The catching protrusion 121 may fit into a catching groove 214 formed in the housing 210. The catching groove 214 may be arranged inside the housing 210 at one side of the housing 210 and may be a groove into which the catching groove 214 may fit.

The catching groove 214 may be a groove recessed in a direction from the inside toward the outside of the housing 210 and may be formed on both sides of the housing 210 with reference to the center of the housing 210. The catching groove 214 may be formed in both the lower plate 212 and the upper plate 213 of the housing 210.

The catching protrusion 121 fits into the catching groove 214, and the lower plate 212 and the upper plate 213 of the housing 210 are coupled to each other, thereby securing the connector 120 to the housing 210. As such, when the connector 120 is secured to the housing 210 via the catching protrusion 121 and the catching groove 214, the connector 120 may be secured even without the use of a pin. Because no pin is used, the flow of the sample moving along the connector 120 may be facilitated, and the efficiency of a test may improve.

Referring to FIGS. 4 and 8, a support 215 having a bar shape may be arranged at one end of the housing 210 to extend from the one end of the housing 210 to the outside of the housing 210.

The support 215 may contact the outside of the connector 120 and may extend from the one end of the housing 210 to the outside of the housing 210 while having a bar shape that has the same width as the width of the connector 120.

The support 215 may be formed on each of the lower plate 212 and the upper plate 213 of the housing 210 and may contact each of a lower surface and an upper surface of the connector 120.

When the length of the connector 120 is large, the connector 120 may be broken due to buckling. The support 215 may be provided to prevent the connector 120 from being broken. The support 215 may support the connector 120 by bringing the support 215 into contact with the connector 120, thereby preventing the connector 120 from being broken.

A test method using the aforementioned diagnostic kit, in which a detection device and a specimen collection tool are integrated, according to an embodiment of the present disclosure, may be described as follows.

The diagnostic kit, in which a detection device and a specimen collection tool are integrated, according to an embodiment of the present disclosure may allow a test to be performed by using a tube 130 containing a developing solution (a diluent or a buffer solution).

Referring to FIG. 2, a specimen is collected from a nasal cavity of a human body by using the specimen collector 110 of the diagnostic kit, in which a detection device and a specimen collection tool are integrated, according to an embodiment of the present disclosure. Next, when the specimen collector 110 is inserted into the tube 130 storing the developing solution, the test may be performed by the device 200, which is connected to the specimen collector 110 via the connector 120.

Specifically, after the specimen is collected by the specimen collector 110, when the specimen collector 110 is inserted into the tube 130, a sample (specimen solution), in which the developing solution is mixed with the specimen, moves to the detection strip 220 of the device 200 through the connector 120.

The sample (specimen solution) having moved to the detection strip 220 is absorbed by the sample pad 221 and thus moves to the conjugation pad 222. When there is a target material in the sample (specimen solution) absorbed by the sample pad 221, the source material in the conjugation pad 222 is coupled with the target material first.

Next, the coupled product moves along the membrane 230 according to the principle of immunochromatography. The coupled product having moved to the membrane 230 meets and is coupled, second, with the source material specifically reacting with the target material, at the position of the test line 231.

When the second coupling occurs at the position of the test line 231, a visible band at the position of the test line 231 is displayed together with the control line 232, and a user may recognize the visible band. The example set forth above is a process when there is the target material in the sample (specimen solution), and the reaction set forth above does not occur when there is no target material in the sample (specimen solution).

The user may recognize that there is the target material in the specimen when the reaction set forth above occurs to form the test line 231, and may recognize that there is no target material in the specimen when the test line 231 is not formed.

Referring to FIG. 2, the housing 210 may include an insertion portion 216, which has a cross-section of a polygonal shape, at one side thereof. As described above, the diagnostic kit, in which a detection device and a specimen collection tool are integrated, according to an embodiment of the present disclosure allows a test to be performed by using the tube 130 containing a developing solution (a diluent or a buffer solution).

Here, during the process of performing the test, the housing 210 is required not to depart from the tube 130. For this purpose, the housing 210 may include the insertion portion 216, which has a cross-section of a polygonal shape.

The insertion portion 216 is a portion that may contact inner side surface of the tube 130, when the housing 210 is inserted into the tube 130. The tube 130 may have a cross-section of a circular shape, and the cross-section of the insertion portion 216 has a polygonal shape, thereby preventing the housing 210 from departing from the tube 130.

More specifically, the insertion portion 216 of the housing 210 may have a quadrangular cross-section, and the insertion portion 216 having the quadrangular cross-section may tightly fit into the tube 130 having a circular cross-section, thereby preventing the housing 210 from departing from the tube 130.

Referring to FIGS. 3 and 4, the diagnostic kit, in which a detection device and a specimen collection tool are integrated, according to an embodiment of the present disclosure may include a cover 240. The cover 240 has an empty space inside thereof and may fit into the housing 210 while covering the specimen collector 110 and the connector 120.

When the diagnostic kit, in which a detection device and a specimen collection tool are integrated, according to an embodiment of the present disclosure is transported, it is needed to prevent the specimen collector 110 and the connector 120 from being contaminated by external contaminant sources.

The cover 240 is provided for this purpose, and because the cover 240 fits into the housing 210 while covering the specimen collector 110 and the connector 120, the specimen collector 110 and the connector 120 may be prevented from being exposed to the outside thereof.

The aforementioned diagnostic kit, in which a detection device and a specimen collection tool are integrated, according to an embodiment of the present disclosure has the following effects.

According to the diagnostic kit, in which a detection device and a specimen collection tool are integrated, according to an embodiment of the present disclosure, a specimen collection process and a testing process may be consecutively performed without separation therebetween by integrating the detection device with the specimen collection tool that may be used for respiratory tests or coronavirus tests.

In this way, the diagnostic kit, in which a detection device and a specimen collection tool are integrated, according to an embodiment of the present disclosure may increase consumer convenience and perform a quick test by reducing a testing operation.

In addition, according to the diagnostic kit, in which a detection device and a specimen collection tool are integrated, according to an embodiment of the present disclosure, because the specimen collection tool is integrated with the detection device, the possibility that an infection source could be leaked from the specimen collection tool may be cut off by discarding the detection device and the specimen collection tool together instead of separately discarding the specimen collection tool.

Furthermore, according to the diagnostic kit, in which a detection device and a specimen collection tool are integrated, according to an embodiment of the present disclosure, because a connector includes a catching protrusion and a housing includes a catching groove to allow the catching protrusion to fit into the catching groove, the flow of a solution moving along the connector may be facilitated.

Heretofore, while the present disclosure has been described with reference to embodiments thereof, it will be understood by those of ordinary skill in the art that these embodiments are provided for illustration only, and that various changes in form and details may be made therein without departing from the spirit and scope of the present disclosure. Therefore, the scope of the present disclosure should be defined only by the accompanying claims and equivalents thereof.

## Claims

1. A diagnostic kit, in which a detection device and a specimen collection tool are integrated, for diagnosing a target material, the diagnostic kit comprising:
a specimen collector capable of collecting a specimen;
a device comprising a housing and a detection strip; and
a connector connecting the specimen collector with the device and moving a solution, the connector having a bar shape.

2. The diagnostic kit of claim 1, wherein the specimen comprises a discharge inside a nasal cavity, and
the diagnostic kit is used for a respiratory infection test or a coronavirus test.

3. The diagnostic kit of claim 1, wherein a head portion of the specimen collector comprises a sponge material.

4. The diagnostic kit of claim 1, wherein an opposite portion of the specimen collector is open, and
the width of the opposite portion of the specimen collector is less than the width of the connector.

5. The diagnostic kit of claim 4, wherein an elastic body is arranged on the opposite portion of the specimen collector.

6. The diagnostic kit of claim 1, wherein the connector comprises a porous material, through which the solution is allowed to move.

7. The diagnostic kit of claim 1, wherein the connector comprises a catching protrusion, which protrudes outward from the connector.

8. The diagnostic kit of claim 7, wherein a catching groove, into which the catching protrusion is allowed to fit, is arranged inside the housing at one side of the housing.

9. The diagnostic kit of claim 1, wherein the housing comprises a support having a bar shape, at one end of the housing, the support extending from the one end of the housing to the outside of the housing and being capable of supporting the connector while contacting the outside of the connector.

10. The diagnostic kit of claim 1, wherein, when the specimen collector is inserted into a tube storing a developing solution, a test is performed by the device connected with the specimen collector via the connector.

11. The diagnostic kit of claim 10, wherein the housing comprises an insertion portion at one side thereof, the insertion portion having a cross-section of a polygonal shape.

12. The diagnostic kit of claim 1, wherein the detection strip of the device comprises:
a sample pad absorbing a sample;
a conjugation pad located under the sample pad and comprising a source material that reacts with the target material; and
a membrane comprising a test line, which is coated with the source material reacting with the target material, and a control line, which is coated with a source material used for a control group.

13. The diagnostic kit of claim 12, wherein the source material is coupled with nano-sized nanoparticles and impregnated into the conjugation pad, and
the nanoparticles comprise one or more of gold particles, silver particles, latex, cellulose, and fluorescent particles.

14. The diagnostic kit of claim 13, wherein the conjugation pad comprises polyester or glass fiber.

15. The diagnostic kit of claim 12, further comprising an absorption pad capable of absorbing the sample that does not react in the conjugation pad and the membrane.

16. The diagnostic kit of claim 12, wherein the target material comprises a material extracted from coronavirus or a material extracted from a respiratory infection source.
